# EUROPEAN PATENT APPLICATION

(11) **EP 4 682 899 A1**
(43) Date of publication of application: **21.01.2026**
(21) Application number: 24189427.8
(22) Date of filing: 18.07.2024
(51) Int. Cl.: G16H 20/00, G16H 50/20, A61B 5/00, A61B 5/20

(54) **RENAL DENERVATION TREATMENT ASSESSMENT USING AMBULATORY BLOOD PRESSURE MONITOR**

(71) Applicant: Koninklijke Philips N.V., 5656 AG Eindhoven (NL)
(72) Inventor: CHEN, Sara Rose, Eindhoven (NL)
(74) Representative: Philips Intellectual Property & Standards

(57) **Abstract**

An apparatus includes a processor configured to receive, via an ambulatory blood pressure monitor, ambulatory blood pressure data for a patient; retrieve, from an electronic health record database, patient data including at least one of demographic data, diagnostic data, or treatment data for the patient; and receive, via an electronic medication diary, medication compliance data for the patient. The processor is also configured to determine a suitability of the patient for a renal denervation treatment, wherein the determination is based on the patient data. The processor is also configured to output, to a display, a screen display based on the determination, wherein the screen display comprises at least one of: a first indication of whether the patient is expected to respond to the renal denervation treatment; or a second indication of an expected level of responsiveness of the patient to the renal denervation treatment.

## Description

### FIELD

The subject matter described herein relates to systems, devices, and methods for determining the suitability and/or expected responsiveness of patients for a renal denervation (RDN) procedure. This RDN suitability prediction system has particular but not exclusive utility for identifying candidates who are likely to respond to a renal denervation procedure.

### BACKGROUND

Uncontrolled high blood pressure is one of the highest causes of death worldwide. Blood pressure is challenging to measure, subject to errors related to the patient, procedure, or equipment. It normally fluctuates throughout the day, and can be raised by up to 26mm Hg even by the patient's presence in a doctor's office (white coat hypertension). As a result, to confirm and better characterize high blood pressure, patients are given ambulatory blood pressure monitors that can trace the patient's blood pressure along the course of their day and night amidst regular activities over several days.

For patients with high blood pressure (typically with consistent systolic values above 130 or diastolic above 80), providers are likely to prescribe lifestyle changes and consider adding blood pressure medication depending on patient risk factors. At above 140 systolic or 90 diastolic, blood pressure medication becomes more common.

About 50% of patients prescribed blood pressure medication are estimated to remain hypertensive despite the medication. This is partially due to lack of effectiveness, and partially due to lack of medication compliance when patients experience unwanted side effects. Alternative and emerging treatment approaches include renal denervation (RDN), a one-time minimally invasive procedure designed to alter the nervous system in a way that provides a meaningful reduction in blood pressure and associated cardio and neurovascular risks.

Recent studies have shown varying levels of efficacy for RDN, with some portion of the population (currently estimated at approximately 30%) showing no response or negative response to the procedure. This demographic of non-responders may present as such for reasons that may stem from a combination of physiological factors and/or inadequately administered therapy. At present, there is no known parameter, algorithm, or method that adequately predicts treatment responders, nor whether a therapy has been applied effectively.

The information included in this Background section of the specification, including any references cited herein and any description or discussion thereof, is included for technical reference purposes only and is not to be regarded as subject matter by which the scope of the disclosure is to be bound.

### SUMMARY

The RDN suitability prediction system of the present disclosure uses an ambulatory blood pressure (BP) monitor with network connectivity to transfer ambulatory BP data for analysis. Behavioral data may be added through permissions from the patient to take medication compliance via an electronic medication diary. Patient identification data allows a link to the patient's electronic health record (EHR) data to extract information such as demographics (age, weight, family history). Outputs include analytic, evidence-based guidance on suitability treatment for treatment such as renal denervation and/or guidance support in medication compliance. In the case of renal denervation, the output can include whether the blood pressure results meet severity levels indicated for the RDN treatment, and what the likelihood is of response and likely level of response if treated.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the RDN suitability prediction system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
**Figure 1** is a schematic, diagrammatic representation, in block diagram form, of at least a portion of an example RDN suitability prediction system, in accordance with aspects of the present disclosure.
**Figure 2** is a schematic, diagrammatic representation, in block diagram form, of at least a portion of an example RDN suitability prediction system, according to aspects of the present disclosure.
**Figure 3** is a schematic, diagrammatic representation, in block diagram form, of an example RDN index calculation process, according to aspects of the present disclosure.
**Figure 4** is a schematic, diagrammatic representation, in block diagram form, of an example calculation process for the weights for an RDN suitability calculation, according to aspects of the present disclosure.
**Figure 5** is a schematic, diagrammatic representation, in block diagram form, of an example machine learning model training process for expected RDN response calculation, according to aspects of the present disclosure.
**Figure 6** is a schematic, diagrammatic representation, in block diagram form, of an example machine learning model training system for RDN index calculation, according to aspects of the present disclosure.
**Figure 7** is a schematic, diagrammatic representation, in block diagram form, of a machine learning inference mode for an example RDN suitability prediction system, according to aspects of the present disclosure.
**Figure 8** is a screen display of an example RDN suitability prediction system, according to aspects of the present disclosure.
**Figure 9** is a schematic diagram of a processor circuit 950, according to aspects of the present disclosure.

### DETAILED DESCRIPTION

In accordance with at least one aspect of the present disclosure, a RDN suitability prediction system is provided which enables a clinician to predict which candidates are likely to show favorable response to RDN treatment, and estimates the response (e.g., in systolic mmHg reduction 6 months post-procedure).

Currently, with renal denervation in an early adopter status, there is room for greater awareness of a patient's suitability for the treatment. To assess a patient, physicians have to collect data from multiple sources, including a standalone ambulatory blood pressure monitor, electronic medical record for their medical history and risk factors, and current patient inputs about their medication compliance. There isn't currently an easy, instant, or consistently repeatable way to combine these factors toward a conclusion and treatment plan.

Combining biometric data, data from the patient's electronic health record, and behavior data collected by the patient can aid in defining the severity of the hypertension and appropriateness of different interventions. An ambulatory 24-hour blood pressure monitor with connectivity enables combination of the blood pressure data with EHR data including demographic, diagnostic and treatment data, and an electronic medication diary to track compliance with current treatment. An analysis application uses those inputs to determine whether a patient's condition reaches a threshold for renal denervation, and also to predict the patient's response to the therapy.

The RDN suitability prediction system of the present disclosure adds wired or wireless network connectivity to an ambulatory blood pressure (BP) monitor, to transfer BP data to an analysis application. The analysis application offers remote display accessible by the health care provider from any Internet connection, and offers clinicians the ability to review the continuous and summary data. Patient identification data in the application allows a link to the patient's electronic health record (EHR) data. The analysis tool extracts relevant inputs such as demographics (age, weight, height, BMI, family history) and combines it with the current blood pressure results.

Behavioral data is optionally added through permissions from the patient to take medication compliance via an electronic on-board medication diary within the patient's blood pressure viewing software or online tools. Outputs include analytic, evidence-based guidance on suitability of the patient for support in medication compliance, or alternative treatment such as renal denervation. In the case of renal denervation, the output can include whether the blood pressure results meet severity levels indicated for the RDN treatment, and what the likelihood is of response and likely level of response if treated.

The present disclosure aids substantially in performing interventional procedures such as renal denervation, by improving the clinician's ability to understand and measure the expected effectiveness of the treatment. Implemented on processors in communication with one or more sensors and one or more databases, the RDN suitability prediction system disclosed herein provides practical detection and measurement of the patient's expected response to the RDN treatment. This improved situational awareness transforms a blind medical procedure with a 70% success rate into one where the success can be accurately predicted, without the normally routine need to try the procedure and then wait to see whether the patient's hypertension declines over period of days or weeks. This unconventional approach improves the functioning of the renal denervation system, by improving the success rate of RDN treatments and by screening out candidates who are not suitable for the procedure or are unlikely to respond favorably to it.

The RDN suitability prediction system may be implemented at least partially as a process viewable on a display, and operated by a control process executing on a processor that accepts user inputs from a keyboard, mouse, or touchscreen interface, and that is in communication with one or more sensors. In that regard, the control process performs certain specific operations in response to different inputs or selections made at different times. Certain outputs of the RDN suitability prediction system may be printed, shown on a display, indicated with lights or tones, or otherwise communicated to human operators. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the RDN suitability prediction system. Certain features may be added, removed, or modified without departing from the spirit of the claimed subject matter.

The present disclosure is related to U.S. Provisional Application No. ___, filed ___, and titled "Catheter-Based Procedures With Procedure Room Detection Of Biomarkers In Patient Blood And Associated Devices, Systems, And Methods" (Atty. Dkt. No. 2023PF00849 / 44755.2399PV01), U.S. Provisional Application No. ___, filed ___, and titled "Multi-Factor Renal Denervation Index For Patient Suitability and/or Expected Responsiveness To Renal Denervation Treatment" (Atty. Dkt. No. 2023PF00861 / 44755.2401PV01), U.S. Provisional Application No. ___, filed ___, and titled "Renal Denervation Treatment Guidance Using Hemodynamic Co-Registration and Associated Systems, Devices, and Methods" (Atty. Dkt. No. 2023PF00857 / 44755.2405PV01), and U.S. Provisional Application No. ___, filed ___, and titled "Renal Nerve Bundle Co-Registration With X-Ray Image For Renal Denervation Treatment Guidance" (Atty. Dkt. No. 2023PF00858 / 44755.2406PV01), each of which is incorporated by reference as though fully set forth herein.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

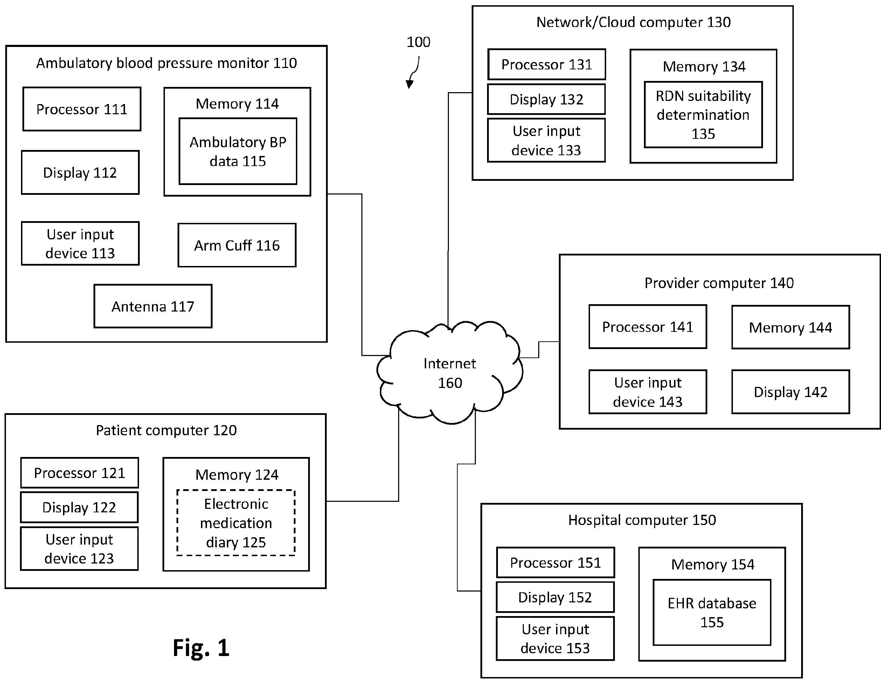

**Figure 1** is a schematic, diagrammatic representation, in block diagram form, of at least a portion of an example RDN suitability prediction system 100, according to aspects of the present disclosure. The RDN suitability prediction system can include an ambulatory blood pressure cuff 110, a patient computer 120, a network/cloud computer 130, a healthcare provider computer 140, and a hospital computer 150, all connected by a network 160 such as the Internet.

In an example, the ambulatory blood pressure monitor 110 includes a processor 111, display 112, user input device 113, a memory 114 containing ambulatory blood pressure data 115, an arm cuff 116, and an antenna 117. In an example, the patient brings the ambulatory blood pressure monitor 110 home and wears it for at least 24 hours, thus providing a full clinical picture of the patient's blood pressure, and whether it is high enough to warrant a diagnosis of hypertension.

In an example, the patient computer 120 includes a processor 121, display 122, user input device 123, and a memory 124 containing an electronic medication diary 125. The patient computer may for example be a personal computer, smartphone, tablet computer, or otherwise, in the possession of the patient. In an example, the electronic medication diary 125 accepts inputs from the user, including inputs regarding the patient's medication compliance (e.g., what doses were taken at what times). The electronic medication diary 125 may for example be a software application running on the patient computer 120, or may be web-accessible by the patient computer 120 while actually running remotely on the network/cloud computer 130..

In an example, the network/cloud computer or manufacturer computer 130 includes a processor 131, display 132, user input device 133, and a memory 134 containing an RDN suitability determination module 135. The network/cloud computer or manufacturer computer 130 may for example be a server, and the RDN suitability determination module 135 may be a web-accessible application running on the server.

In an example, the healthcare provider computer 140 includes a processor 141, display 142, user input device 143, and memory 144. The healthcare provider computer 140 may for example be a personal computer, smartphone, tablet computer, etc., accessible by the clinician who is making the decision as to whether to perform RDN therapy, and may run a web browser or other application capable of sending data to, and receiving screen displays from, the RDN suitability determination module 135 running on the network/cloud computer 130.

In an example, the hospital computer 150 may include a processor 151, display 152, user input device 153, and a memory 154 containing an electronic health records (EHR) database. The EHR database may for example include patient data such as the patient's medical history, vital signs, demographics (age, weight, etc.), and what medications are prescribed to the patient.

Block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available to the user. Similarly, block diagrams may show a particular arrangement of components, modules, services, steps, processes, or layers, resulting in a particular data flow. It is understood that some aspects of the systems disclosed herein may include additional components, that some components shown may be absent from some aspects, and that the arrangement of components may be different than shown, resulting in different data flows while still performing the methods described herein.

Before continuing, it should be noted that the examples described above are provided for purposes of illustration, and are not intended to be limiting. Other devices and/or device configurations may be utilized to carry out the operations described herein.

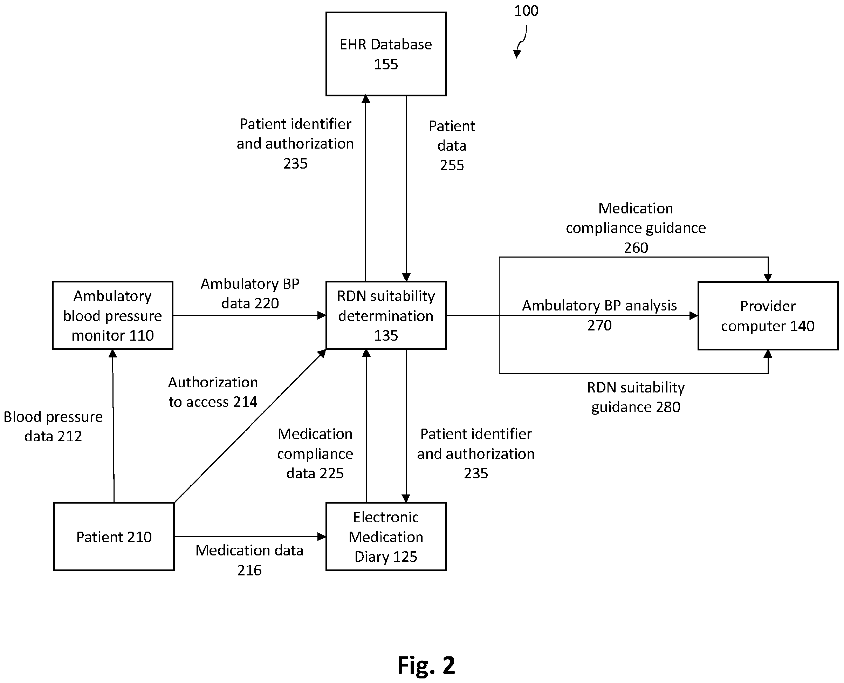

**Figure 2** is a schematic, diagrammatic representation, in block diagram form, of at least a portion of an example RDN suitability prediction system 100, according to aspects of the present disclosure. In the example shown in Figure 2, the patient 210 provides blood pressure data 212 to the ambulatory blood pressure monitor 110, provides access authorization 214 to the RDN suitability determination module 135, and provides medication data 216 to the electronic medication diary 125. The ambulatory blood pressure monitor 110 provides ambulatory BP data 220 to the RDN suitability determination module 135.

The RDN suitability determination module 135 provides the patient identifier and authorization to the electronic medication diary 125 and the EHR database 155.

Once authorized, the electronic medication diary 125 provides medication compliance data 225 to the RDN suitability determination module 135. Once authorized, the EHR database 155 provides patient data 255 to the RDN suitability determination module 135.

In the example shown in Figure 2, the RDN suitability determination module 135 provides several things to the provider computer 140. First is medication compliance guidance 260, indicating for example what dosage the patient is achieving, whether and when the patient is missing medication doses, whether the patient may benefit from guidance or coaching to increase medication compliance, etc. Second is ambulatory BP analysis 270, indicating for example whether the patient meets the clinical criteria for a diagnosis of hypertension severe enough that RDN should be considered. In an example, a systolic blood pressure (SBP) of 140 or greater may warrant consideration of RDN treatment. Third is RDN suitability guidance 280. The RDN suitability guidance 280 may for example include a yes/no indication of whether the patient is expected to respond to RDN treatment (e.g., expected to show a systolic blood pressure (SBP) reduction of 5 mmHg or greater), and/or a categorization of the patient's expected response (e.g., low, medium, or high response), and/or a numerical value indicating the patient's expected SBP reduction in mmHg.

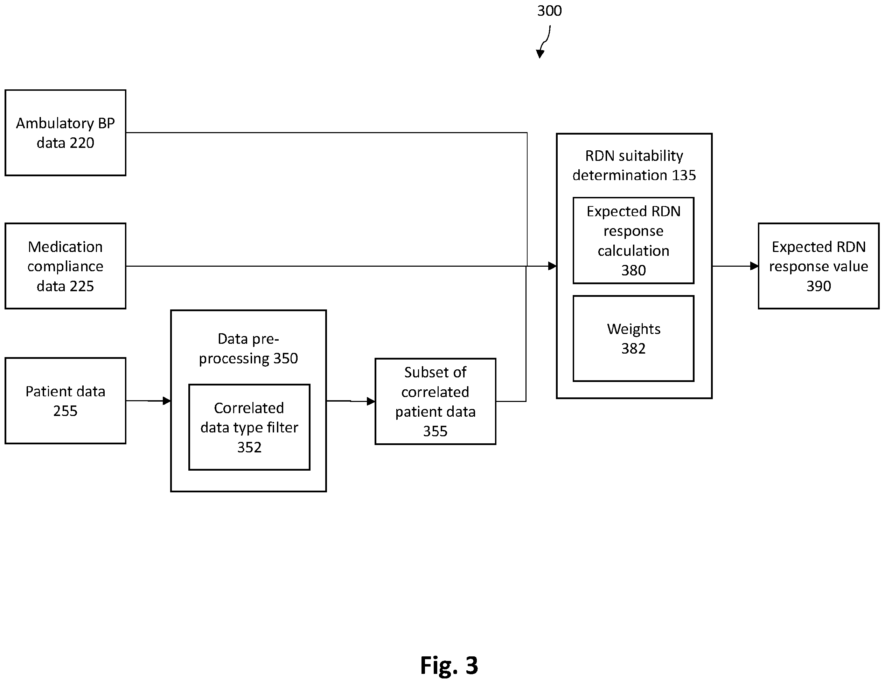

**Figure 3** is a schematic, diagrammatic representation, in block diagram form, of an example RDN index calculation process 300, according to aspects of the present disclosure. The calculation process 300 begins with the ambulatory BP data 220, medication compliance data 225, and patient data 255. In the example shown in Figure 3, the ambulatory BP data 220 and medication compliance data 225 are sent directly to the RDN suitability determination module 135. However, the patient data 255 is first sent to a preprocessing step 350, which uses a correlated data type filter 352 to screen out any patient data 255 that is not pertinent to determining whether to perform RDN. The data pre-processing step 350 yields a subset of correlated patient data 355 (e.g., only the patient data thought to be significant predictors or RDN success), which is then received by the RDN suitability determination module 135.

The RDN suitability determination module 135 includes an expected RDN response calculation 380. In some aspects, the expected RDN response calculation may be a simple arithmetic combination of the input metrics, such as a sum or product. In other aspects, the expected RDN response calculation 380 may rely on weights 382 that are applied to the input metrics. For example, the weights 282 may quantify the relative importance of different input metrics such that, for example, mean blood pressure (with an exemplary weight of 4.0) may be twice as important as patient age (with an exemplary weight of 2.0) and four times as important as BMI (with a weight of 1.0) for the accurate prediction of a patient's response to the RDN procedure. Weights 382 may for example be developed based on clinical research, medical literature, standards set by physician's organizations, clinical trials, statistical analysis, and otherwise. In some cases, for negative correlations, a weight may be less than zero.

The output of the expected RDN response calculation 380 is expected RDN response value 390, which may for example be a continuous value between 0.0 and 1.0, a classification value (e.g., low response, medium response, high response), a binary yes/no value, or a value with no specific upper or lower bounds, such as a predicted amount of systolic blood pressure reduction (e.g., in mmHg), that is nevertheless representative of the patient's suitability for, and probable response to, the RDN procedure.

The RDN suitability determination module 135 may, in some aspects, employ a supervised learning approach, wherein a trained machine learning model (e.g., an artificial neural network, ensemble learning (e.g., random forest), linear regression, logistic regression, etc.) receives the input data 220, 225, 355 and generates the expected RDN response value 390.

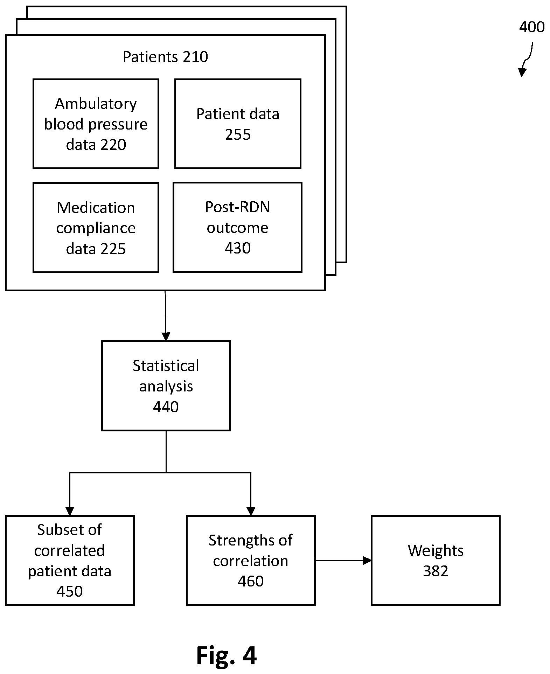

**Figure 4** is a schematic, diagrammatic representation, in block diagram form, of an example calculation process 400 for the weights 382 for an RDN suitability calculation, according to aspects of the present disclosure. The calculation process 400 begins with ambulatory blood pressure data 220, medication compliance data 225, patient data 255, and post-RDN outcomes 430 (e.g., measured systolic blood pressure reduction) for a population of patients 210. These values 220, 225, 255, 430 serve as inputs to a statistical analysis 440 that yields a list of correlated patient parameters 450, e.g., a list of those metrics 220, 225, 255 that show a statistically significant correlation with the post-RDN outcomes 430. The statistical analysis may for example include linear or non-linear regression of multiple independent variables in search of the strongest correlation to the post-RDN outcomes 430. The statistical analysis also yields the strengths 460 of these correlations, which can then either serve as weights 482 or can be used to calculate the weights 482. The correlation strengths 460 can also be used to determine the correlated data type filter 352 of Figure 3.

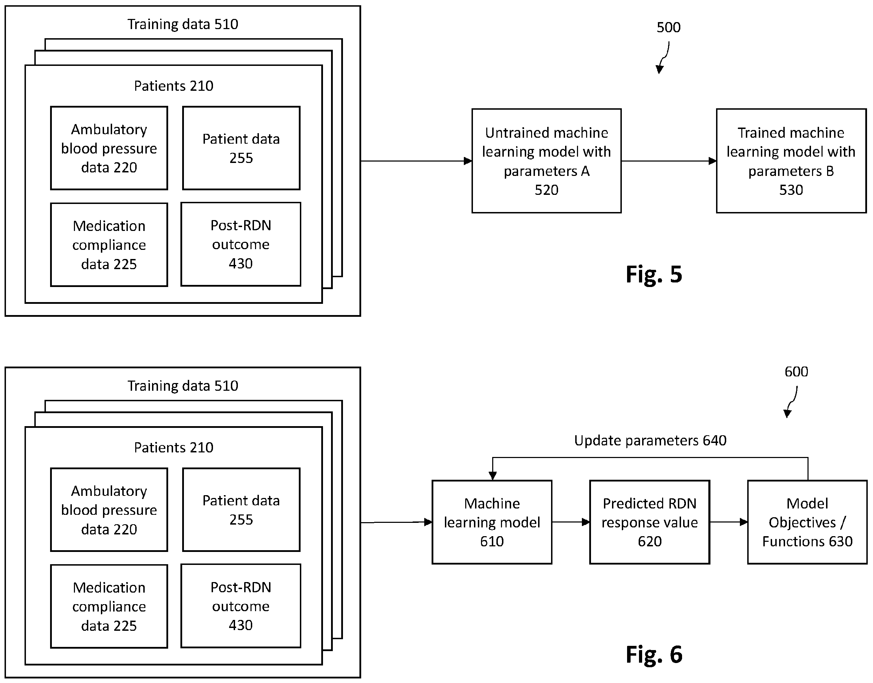

**Figure 5** is a schematic, diagrammatic representation, in block diagram form, of an example machine learning model training process 500 for expected RDN response calculation, according to aspects of the present disclosure. The training process 500 begins with a set of training data 510 that includes ambulatory blood pressure data 220, medication compliance data 225, patient data 255, and post-RDN outcomes 430 (e.g., measured systolic blood pressure reduction) for a population of patients 210. These values 220, 225, 255, 430 are used to train an untrained machine learning model 520 with parameters A, to produce a trained machine learning model 530 with parameters B, that can be used in inference mode to calculate an expected RDN response for a patient based on that patient's metrics 220, 225, and 255. The machine learning model may for example use supervised learning based on multivariate regression models.

**Figure 6** is a schematic, diagrammatic representation, in block diagram form, of an example machine learning model training system 600 for RDN index calculation, according to aspects of the present disclosure. The training 600 begins with a set of training data 510 that includes ambulatory blood pressure data 220, medication compliance data 225, patient data 255, and post-RDN outcomes 430 (e.g., measured systolic blood pressure reduction) for a population of patients 210. These values 220, 225, 255, 430 are used to train a predictive machine learning network or model 610, which produces predicted RDN response values 620 for each patient 210. These predicted RDN response values 620 are then evaluated against the model's objectives or functions 630 (e.g., a difference between the post-RDN outcome 430 and the predicted RDN response value 620) to determine the success of the training, and, if the predicted RDN response values 620 fall below a threshold of desired accuracy (e.g. for predicting the post-RDN outcomes 430), the parameters go through repeated updates 640 until the desired accuracy is achieved. In some instances, updating 640 may be accomplished using gradients of the objective functions and backpropagation to update the parameters of the predictive network 610. In some aspects, retraining and/or fine tuning can be done for newly introduced patient data (e.g., within clinical trials).

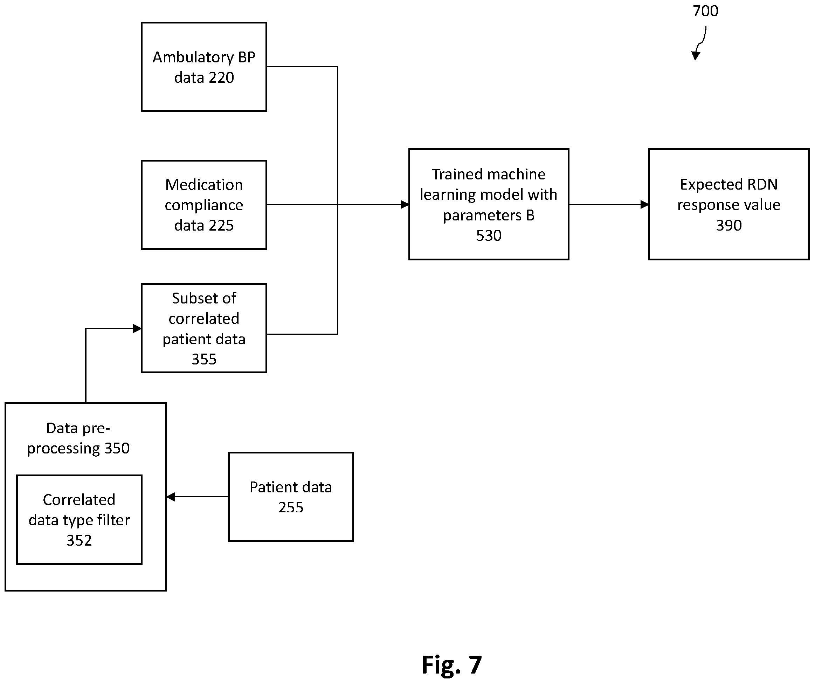

**Figure 7** is a schematic, diagrammatic representation, in block diagram form, of a machine learning inference mode 700 for an example RDN suitability prediction system, according to aspects of the present disclosure. In the example shown in Figure 7, patient data 255 goes through a pre-processing step 350 including correlated data type filtering 352, to yield a subset of correlated patient data 355. The subset of correlated patient data 355, along with the ambulatory blood pressure data 220 and the medication compliance data 225, is then fed to the trained machine learning model 530, which produces the expected RDN response value 390 as an output.

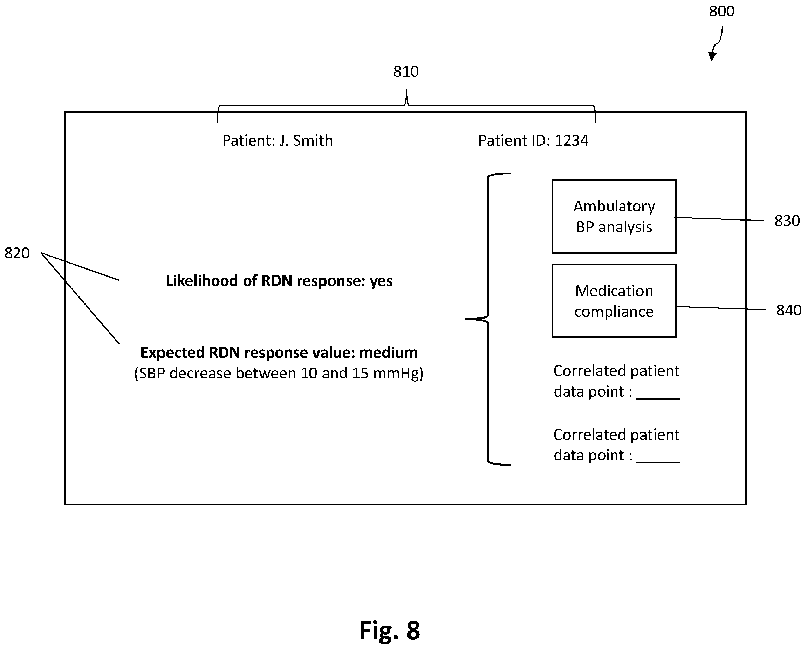

**Figure 8** is a screen display 800 of an example RDN suitability prediction system, according to aspects of the present disclosure. The screen display 800 includes patient information 810, and an RDN expected response value display 820. The expected response value display can include one or more of a binary yes/no value, a categorization value (e.g., low response, medium response, high response), or a numerical value (e.g., expected BP reduction in mmHg). In the example shown in Figure 8, to help clinicians understand the derivation and meaning of the RDN expected response, the screen display 800 also includes an ambulatory BP analysis button 830, which may for example generate a graph or pop-up window with details about the patient's blood pressure, thresholds for hypertension and RDN treatment candidacy, etc. The screen display 800 also includes a medication compliance button 840, which may for example generate a graph or pop-up window with details about the patient's medication compliance, doses taken, doses skipped, etc. The screen display 800 also includes correlated patient data 850, that can help the clinician to understand the expected response value.

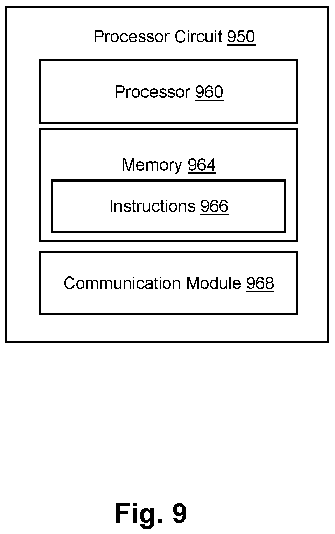

**Figure 9** is a schematic diagram of a processor circuit 950, according to aspects of the present disclosure. The processor circuit 950 may be implemented in system, or apparatus 100, processor 111, processor 121, processor 131, processor 141, processor 151, or other devices or workstations (e.g., third-party workstations, network routers, etc.), or on a cloud processor or other remote processing unit, as necessary to implement the method. As shown, the processor circuit 950 may include a processor 960, a memory 964, and a communication module 968. These elements may be in direct or indirect communication with each other, for example via one or more buses.

The processor 960 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 960 may also comprise another hardware device, a firmware device, or any combination thereof configured to perform the operations described herein. The processor 960 may also be implemented as a combination of computing devices, e.g., a combination of a DSP and a microprocessor, a plurality of microprocessors, one or more microprocessors in conjunction with a DSP core, or any other such configuration.

The memory 964 may include a cache memory (e.g., a cache memory of the processor 960), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an aspect, the memory 964 includes a non-transitory computer-readable medium. The memory 964 may store instructions 966. The instructions 966 may include instructions that, when executed by the processor 960, cause the processor 960 to perform the operations described herein. Instructions 966 may also be referred to as code. The terms "instructions" and "code" should be interpreted broadly to include any type of computer-readable statement(s). For example, the terms "instructions" and "code" may refer to one or more programs, routines, subroutines, functions, procedures, etc. "Instructions" and "code" may include a single computer-readable statement or many computer-readable statements.

The communication module 968 can include any electronic circuitry and/or logic circuitry to facilitate direct or indirect communication of data between the processor circuit 950, and other processors or devices. In that regard, the communication module 968 can be an input/output (I/O) device. In some instances, the communication module 968 facilitates direct or indirect communication between various elements of the processor circuit 950 and/or the system 100. The communication module 968 may communicate within the processor circuit 950 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture

(ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, data sharing between the processor and central server, or readings from the sensors) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

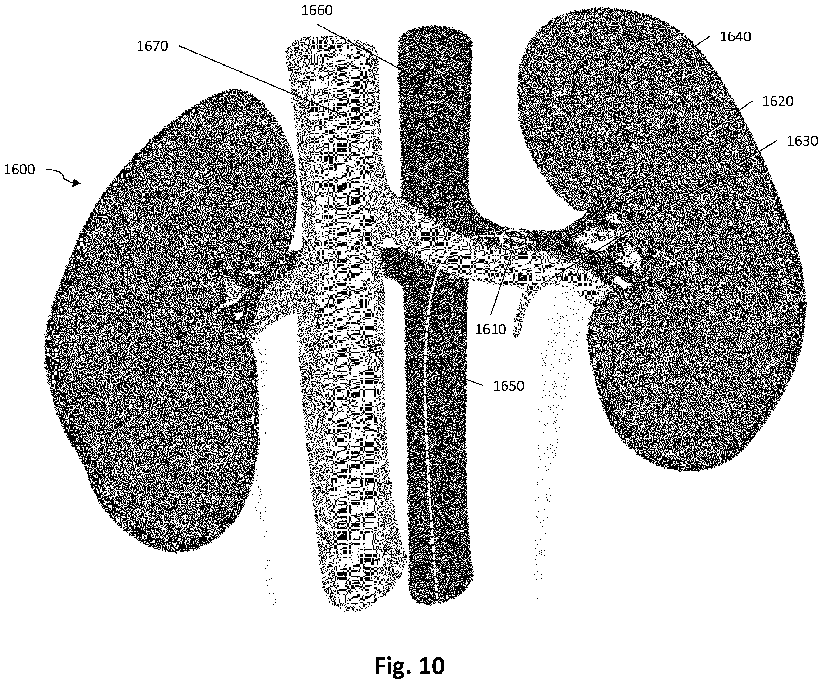

**Figure 10** is a schematic, diagrammatic representation of the renal vasculature 1600 of a patient, with a renal denervation treatment device 1610 in the left renal artery 1620 of the left kidney 1640, according to aspects of the present disclosure. In an example, the renal denervation treatment device 1610 may include a catheter 1650 with a renal denervation tool (e.g., electrodes, balloon, etc.) that delivers energy to injure the renal nerves, in order to lower the patient's blood pressure. Depending on the implementation, the delivered energy may be electrical energy, chemical energy, heat, cold, etc. The renal denervation treatment device 1610 may for example enter the renal artery 1620 via the abdominal aorta 1660. Also visible is the renal vein 1630. The renal denervation can be performed based on the guidance described herein.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an apparatus that includes a processor configured to: receive, via an ambulatory blood pressure monitor, ambulatory blood pressure data for a patient; retrieve, from an electronic health record database, at least one of demographic data, diagnostic data, or treatment data for the patient; receive, via an electronic medication diary, medication compliance data for the patient; determine a suitability of the patient for a renal denervation treatment, where the determination is based on the ambulatory blood pressure data, at least one of the demographic data, the diagnostic data, or the treatment data, and the medication compliance data; and output, to a display, a screen display based on the determination, where the screen display may include at least one of: a first indication of whether the patient is expected to respond to the renal denervation treatment; or a second indication of an expected level of responsiveness of the patient to the renal denervation treatment. Other examples of this aspect include corresponding computer systems, apparatus, and computer programs recorded on one or more computer storage devices, each configured to perform the actions of the methods.

Implementations may include one or more of the following features. In some aspects, the suitability of the patient for a renal denervation treatment may include the first indication of whether the patient is expected to respond to the renal denervation treatment. In some aspects, the suitability of the patient for a renal denervation treatment may include the second indication of an expected level of responsiveness of the patient to the renal denervation treatment. In some aspects, the at least one of demographic data, diagnostic data, or treatment data for the patient may include at least one of an age, weight, height, BMI, or family history of the patient. In some aspects, the medication compliance data includes at least one of doses taken or doses missed. In some aspects, the screen display further may include at least one of patient data, correlated patient data, an ambulatory blood pressure analysis, or the medication compliance data. In some aspects, determining the suitability of the patient for the renal denervation treatment involves filtering out portions of the demographic data, the diagnostic data, or the treatment data that have a low correlation with the suitability of the patient for the renal denervation treatment. In some aspects, determining the suitability of the patient for the renal denervation treatment involves applying weights to the ambulatory blood pressure data, the demographic data, the diagnostic data, the treatment data, or the medication compliance data. In some aspects, the weights are determined based on a statistical analysis of a correlation of the ambulatory blood pressure data, the demographic data, the diagnostic data, the treatment data, or the medication compliance data with the suitability of the patient for the renal denervation treatment. In some aspects, determining the suitability of the patient for the renal denervation treatment involves a trained neural network. In some aspects, the trained neural network is trained using supervised learning based on multivariate regression models. In some aspects, the medication compliance data is based on inputs by the patient into a patient computer. The demographic data, the diagnostic data, or the treatment data is based on an electronic health record stored on a hospital computer. In some aspects, determining the suitability of the patient for the renal denervation treatment is performed on a network computer accessible to a healthcare provider computer via a network. Implementations of the described techniques may include hardware, a method or process, or computer software on a computer-accessible medium.

One general aspect includes a method. The method includes receiving, with a processor, ambulatory blood pressure data for a patient via an ambulatory blood pressure monitor; retrieving, with the processor at least one of demographic data, diagnostic data, or treatment data for the patient from an electronic health record database; receiving, with the processor, medication compliance data for the patient via an electronic medication diary; determining, with the processor, a suitability of the patient for a renal denervation treatment, where determining is based on the ambulatory blood pressure data, at least one of the demographic data, the diagnostic data, or the treatment data, and the medication compliance data; and outputting, to a display, a screen display based on the determining, where the screen display may include at least one of: a first indication of whether the patient is expected to respond to the renal denervation treatment; or a second indication of an expected level of responsiveness of the patient to the renal denervation treatment. Other examples of this aspect include corresponding computer systems, apparatus, and computer programs recorded on one or more computer storage devices, each configured to perform the actions of the methods.

Accordingly, it can be seen that the RDN suitability prediction system advantageously provides a means for determining, in near-real-time in advance of a procedure, a patient's suitability for, and likely response to, a renal denervation procedure.

A number of variations are possible on the examples and aspects described above. For example, other variables may be used than those listed herein, and other sensors or sensor types may be employed. The technology described herein may be used not only before medical interventions, but also at other times, to provide metrics that may be indicative of a health state or disease state of the patient..

Accordingly, the logical operations making up the aspects of the technology described herein are referred to variously as operations, steps, objects, elements, components, or modules. Furthermore, it should be understood that these may occur, or be performed or arranged, in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the RDN suitability prediction system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the RDN suitability prediction system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

Additional embodiments
Embodiment 1. An apparatus, comprising:
   a processor configured to:
   receive, via an ambulatory blood pressure monitor, ambulatory blood pressure data for a patient;
   retrieve, from an electronic health record database, at least one of demographic data, diagnostic data, or treatment data for the patient;
   receive, via an electronic medication diary, medication compliance data for the patient;
   determine a suitability of the patient for a renal denervation treatment, wherein the determination is based on the ambulatory blood pressure data, at least one of the demographic data, the diagnostic data, or the treatment data, and the medication compliance data; and
   output, to a display, a screen display based on the determination, wherein the screen display comprises at least one of:
      a first indication of whether the patient is expected to respond to the renal denervation treatment; or
      a second indication of an expected level of responsiveness of the patient to the renal denervation treatment.
Embodiment 2. The apparatus of Embodiment 1, wherein the suitability of the patient for a renal denervation treatment comprises the first indication of whether the patient is expected to respond to the renal denervation treatment.
Embodiment 3. The apparatus of Embodiment 1, wherein the suitability of the patient for a renal denervation treatment comprises the second indication of an expected level of responsiveness of the patient to the renal denervation treatment.
Embodiment 4. The apparatus of Embodiment 1, wherein the at least one of demographic data, diagnostic data, or treatment data for the patient comprises at least one of an age, weight, height, body mass index (BMI), or family history of the patient.
Embodiment 5. The apparatus of Embodiment 1, wherein the medication compliance data includes at least one of doses taken or doses missed.
Embodiment 6. The apparatus of Embodiment 1, wherein the screen display further comprises at least one of patient data, correlated patient data, an ambulatory blood pressure analysis, or the medication compliance data.
Embodiment 7. The apparatus of Embodiment 1, wherein determining the suitability of the patient for the renal denervation treatment involves filtering out portions of the demographic data, the diagnostic data, or the treatment data that have a low correlation with the suitability of the patient for the renal denervation treatment.
Embodiment 8. The apparatus of Embodiment 1, wherein determining the suitability of the patient for the renal denervation treatment involves applying weights to the ambulatory blood pressure data, the demographic data, the diagnostic data, the treatment data, or the medication compliance data.
Embodiment 9. The apparatus of Embodiment 8, wherein the weights are determined based on a statistical analysis of a correlation of the ambulatory blood pressure data, the demographic data, the diagnostic data, the treatment data, or the medication compliance data with the suitability of the patient for the renal denervation treatment.
Embodiment 10. The apparatus of Embodiment 1, wherein determining the suitability of the patient for the renal denervation treatment involves a trained neural network.
Embodiment 11. The apparatus of Embodiment 1, wherein the trained neural network is trained using supervised learning based on multivariate regression models.
Embodiment 12. The apparatus of Embodiment 1, wherein the medication compliance data is based on inputs by the patient into a patient computer.
Embodiment 13. The apparatus of Embodiment 1, wherein the demographic data, the diagnostic data, or the treatment data is based on an electronic health record stored on a hospital computer.
Embodiment 14. The apparatus of Embodiment 1, wherein determining the suitability of the patient for the renal denervation treatment is performed on a network computer accessible to a healthcare provider computer via a network.
Embodiment 15. A method, comprising:
   receiving, with a processor, ambulatory blood pressure data for a patient;
   retrieving, with the processor, at least one of demographic data, diagnostic data, or treatment data for the patient from an electronic health record database;
   receiving, with the processor, medication compliance data for the patient via an electronic medication diary;
   determining, with the processor, a suitability of the patient for a renal denervation treatment, wherein determining is based on the ambulatory blood pressure data, at least one of the demographic data, the diagnostic data, or the treatment data, and the medication compliance data; and
   outputting, to a display, a screen display based on the determining, wherein the screen display comprises at least one of:
      a first indication of whether the patient is expected to respond to the renal denervation treatment; or
      a second indication of an expected level of responsiveness of the patient to the renal denervation treatment

## Claims

1. An apparatus, comprising:
a processor configured to:
receive ambulatory blood pressure data for a patient;
retrieve, from an electronic health record database, at least one of demographic data, diagnostic data, or treatment data for the patient;
receive, via an electronic medication diary, medication compliance data for the patient;
determine a suitability of the patient for a renal denervation treatment, wherein the determination is based on the ambulatory blood pressure data, at least one of the demographic data, the diagnostic data, or the treatment data, and the medication compliance data; and
output, to a display, a screen display based on the determination, wherein the screen display comprises at least one of:
a first indication of whether the patient is expected to respond to the renal denervation treatment; or
a second indication of an expected level of responsiveness of the patient to the renal denervation treatment.

2. The apparatus of claim 1, wherein the suitability of the patient for a renal denervation treatment comprises the first indication of whether the patient is expected to respond to the renal denervation treatment.

3. The apparatus of claim 1 or 2, wherein the suitability of the patient for a renal denervation treatment comprises the second indication of an expected level of responsiveness of the patient to the renal denervation treatment.

4. The apparatus of any one of the previous claims, wherein the at least one of demographic data, diagnostic data, or treatment data for the patient comprises at least one of an age, weight, height, body mass index (BMI), or family history of the patient.

5. The apparatus of any one of the previous claims, wherein the medication compliance data includes at least one of doses taken or doses missed.

6. The apparatus of any one of the previous claims, wherein the screen display further comprises at least one of patient data, correlated patient data, an ambulatory blood pressure analysis, or the medication compliance data.

7. The apparatus of any one of the previous claims, wherein determining the suitability of the patient for the renal denervation treatment involves filtering out portions of the demographic data, the diagnostic data, or the treatment data that have a low correlation with the suitability of the patient for the renal denervation treatment.

8. The apparatus of any one of the previous claims, wherein determining the suitability of the patient for the renal denervation treatment involves applying weights to the ambulatory blood pressure data, the demographic data, the diagnostic data, the treatment data, or the medication compliance data, wherein the weights are determined based on a statistical analysis of a correlation of the ambulatory blood pressure data, the demographic data, the diagnostic data, the treatment data, or the medication compliance data with the suitability of the patient for the renal denervation treatment.

9. The apparatus of any one of the previous claims, wherein determining the suitability of the patient for the renal denervation treatment involves a trained neural network.

10. The apparatus of any one of the previous claims, wherein the medication compliance data is based on inputs by the patient into a patient computer, wherein the demographic data, the diagnostic data, or the treatment data is based on an electronic health record stored on a hospital computer.

11. The apparatus of any one of the previous claims, wherein determining the suitability of the patient for the renal denervation treatment is performed on a network computer accessible to a healthcare provider computer via a network.

12. A method, comprising:
receiving, with a processor, ambulatory blood pressure data for a patient;
retrieving, with the processor, at least one of demographic data, diagnostic data, or treatment data for the patient from an electronic health record database;
receiving, with the processor, medication compliance data for the patient via an electronic medication diary;
determining, with the processor, a suitability of the patient for a renal denervation treatment, wherein determining is based on the ambulatory blood pressure data, at least one of the demographic data, the diagnostic data, or the treatment data, and the medication compliance data; and
outputting, to a display, a screen display based on the determining, wherein the screen display comprises at least one of:
a first indication of whether the patient is expected to respond to the renal denervation treatment; or
a second indication of an expected level of responsiveness of the patient to the renal denervation treatment; and
optionally, controlling a display to output the screen display to a user.

13. The method of claim 12, wherein determining the suitability of the patient for the renal denervation treatment involves a trained neural network.

14. A computer program comprising computer readable instructions which, when executed, cause implementation of the method of claim 12 or 13.

15. A method of providing the computer program of claim 14 having instructions for causing implementation of claim 13 starting from a computer program comprising instructions encoding at least an untrained neural network, the method comprising training of the untrained neural network to provide the trained neural network.
